# EUROPEAN PATENT APPLICATION

(11) **EP 3 871 653 A1**
(43) Date of publication of application: **01.09.2021**
(21) Application number: 20159724.2
(22) Date of filing: 27.02.2020
(51) Int. Cl.: A61K 8/04, A61K 8/34, A61K 8/92, A61K 8/99, A61Q 15/00

(54) **A COMPOSITION CONTAINING A VITAL PROBIOTIC CULTURE FOR HUMAN OR VETERINARY USE AS A DEODORANT**

(71) Applicant: MicroCen Trans s.r.o, 602 00 Brno (CZ)
(72) Inventor: RYSAVKA, Petr, 683 03 Nemojany (CZ); PRUS, Libor, 735 72 ./.Petrovice u Karviné (CZ)
(74) Representative: Brinkmann & Partner

(57) **Abstract**

The composition of the invention contains a live probiotic culture in dried vital form, stable in the long term in a solution of at least 98% alcohol, preferably encapsulated in an oil component. The composition is suitable for human or veterinary use as a deodorantcontaining a perfuming ingredient, especially in a spray with an inert propellant gas, preferably nitrogen.

## Description

### Field of the invention

The invention relates to a composition containing a vital probiotic culture for human or veterinary use as a deodorant.

### Background of the invention

Body odour plays an important role in the animal kingdom. In animals it can serve to deter predators or signal that prey is inedible. Certain animals feigning death produce odours reminiscent of bodily decay to give the impression that they have been dead for a long time. Body odour in humans is considered undesirable and unpleasant in most cultures, in spite of the fact that it plays an important role in determining the genetic compatibility of individuals and, therefore, the selection of a suitable partner. The most important source of body odour in humans is the armpit.

Many apocrine and exocrine sebaceous glands are found in the armpit. These produce various sweat secretions, such as long-chain fatty acids, fatty acids linked with amino acids, sulphur-containing amino acids and hormones - these molecules are too large to be volatile or cause an odour. An odour is caused by the action of bacteria present on the skin (5). Certain bacteria live on these sweat secretions and cause their decomposition into smaller molecules that do cause an odour (steroids, short-chain and medium-chain fatty acids (C2-C10) and, most importantly, thio-alcohols, of which 3-methyl-3-sulfanylhexan-1-ol (3M3SH) has been identified as the primary cause of odour).

Since the armpit is an environment markedly different from other parts of the body surface, in terms of its humidity, temperature and pH for example, the composition of the armpit microbiome (the microorganisms living in the armpit) is also highly specific. The predominant bacterial types are *Corynebacterium, Staphylococcus* and *Propionibacterium.* Bacteria of the genera *Corynebacterium* and *Staphylococcus* (2) contribute most to body odour. The greater the proportion of aerobic lipophilic Corynebacteria in the armpit, the stronger the odour. Research has also shown that *Staphylococcus hominis* is responsible for the production of the strong smelling product 3M3SH.

The traditional way of fighting body odour is the use of deodorants or antiperspirants, which promise to reduce the number of undesirable bacteria and eliminate, or at least reduce, body odour. This approach may, however, be a double-edged sword. Studies have shown that although the use of deodorants may lead to a reduction in the number of bacteria in the armpit, the final result when deodorant use is discontinued is an increase in diversity and, first and foremost, a significant change in the representation of individual bacterial species (3). A reduced density of bacteria when deodorant use is discontinued provides an opportunity for colonisation by new species. The result in a number of people is excessive colonisation of the armpit by bacteria producing malodourous metabolites and an odour even worse than that before deodorant was used in the first place, as this involves facultative aerobic bacteria capable of surviving inside the sweat glands or in the hair follicles. The rapid reproduction and dominance of these bacteria occurs when deodorant use is discontinued. Bacteria that do not produce an odour, such as *Staphylococcus epidermidis* and *Propionibacterium acnes,* compete under normal circumstances with odour-producing bacteria, reduce them in number and thereby reduce the odour. These are, however, aerobic bacteria that live on the surface of the skin - their renewal after deodorant use is discontinued is considerably more difficult than the renewal of facultative aerobic bacteria that are hidden so some extent from the action of deodorant (7). Body odour can, for this reason, be fought with the use of deodorants only by means of their continual application. Certain substances used in antiperspirants may even increase the risk of oncological diseases (1).

Various approaches have begun to appear in recent years that rather than merely eliminate the armpit microbiome (using deodorants or antibiotic) focus on its promotion with an emphasis on bacteria that do not produce an odour (4). It has been found that a greater occurrence of the bacteria *Staphylococcus epidermidis* and *Propionibacterium acnes* is associated with a more pleasant body smell. The use of probiotics/prebiotics/synbiotics, i.e. live microbial cultures, their lysates or a combination of both, seems to be a possible solution to achieving a permanent reduction in body odour.

The use of probiotics and prebiotics for the targeted enhancement of the population of bacteria that do not produce an odour may be an alternative. Probiotics applied locally to the skin may have a positive action due to competition with harmful microorganisms, may secrete useful metabolites, reduce the pH of the skin, or reinforce the barrier function of the skin against harmful external factors. Probiotics have already shown themselves to be effective against other dermatological conditions, such as atopic eczema, acne, burns and dry skin (6).

Suitably chosen probiotics can lead to an increased proportion of bacteria that do not produce odour, while prebiotics selectively promote their growth. Bacteria that do not produce an odour, such as *Staphylococcus epidermidis* and *Propionibacterium acnes,* compete with bacteria that do produce odour and eliminate, or at least limit, their growth. Prebiotics/probiotics may be applied in the form of a deodorant spray in the way the regular user is accustomed to without it being necessary to undergo any kind of further procedure. Selective support of "the right bacteria" and inhibition of "bad bacteria" can lead not only to the short-term elimination of odour during the period of use of the deodorant, but also to long-term improvement to body odour.

There are already several deodorants with probiotics on the market today. One of these products is Honestly Phresh Deodorant in a Plumérie Monoi spray. Nevertheless, this product does not contain live probiotics, but merely inactivated ferments. Another of the products available is Mother Dirt - a probiotic in a spray. It contains Nitrosomonas eutropha, an AOB (ammonia-oxidising bacteria, not further specified). This is not, however, a spray in a pressure vessel. It does not contain ethanol and does not declare a defined quantity of bacteria. It also does not target lactic acid bacteria in any way. Another product is the probiotic deodorant From Molly With Love. This does not, however, contain live probiotics and is not a deodorant in a spray, but a solid deodorant. All Natural Probiotic Deodorant does not contain live bacteria. It states probiotics, though these are inactivated, and merely the number of bacterial cells added to the product. Another product, Dirty Stash Bath Co, a probiotic deodorant not containing live stable probiotics, comes in a spray for convenient use.

US2014004070 presents a composition for use in cosmetics or pharmacy containing microorganisms that are capable of suppressing production of odorous compounds, in particular 3-methyl-2-hexenoic acid or its derivates, by means of axillary bacteria. Patent application WO 2016130983 describes a deodorant in which there are probiotic bacteria not fermenting sweat in dry form that are humidified after application to the skin. The subject of document US4871539 is a deodorant containing probiotic strains of Lactobacillus and Streptococcus producing an antibiotic. Probiotic cultures of a disinfectant composition according to document US2017281695 limit the binding of pathogens to the skin.

The current designs of deodorants represent limited solutions, generally based on the use of non-living bacteria. They do not resolve the issue of long-term maintenance of viable bacteria in a preparation that can act during long-term deodorant use on the formation and maintenance of the natural colonisation of the skin by aerobic bacteria that do not produce an odour.

### References

1. Mohamed Farouk Allam. 2016. "Breast Cancer and Deodorants / Antiperspirants: A Systematic Review." Central European Journal of Public Health 24(3):245-47.
2. Daniel Bawdon, Diana S. Cox, David Ashford, A. Gordon James and Gavin H. Thomas. 2015. "Identification of Axillary Staphylococcus Sp. Involved in the Production of the Malodorous Thioalcohol 3-Methyl-3-Sulfanylhexan-1-ol." FEMS Microbiology Letters 362(16):1-10.
3. Chris Callewaert, Prawira Hutapea, Tom Van de Wiele and Nico Boon. 2014. "Deodorants and Antiperspirants Affect the Axillary Bacterial Community." Archives of Dermatological Research 306(8):701-10.
4. Chris Callewaert, Jo Lambert and Tom Van de Wiele. 2017. "Towards a Bacterial Treatment for Armpit Malodour." Experimental Dermatology 26(5):388-91.
5. A. Gordon James, Corrine J. Austin, Diana S. Cox, David Taylor and Ralph Calvert. 2013. "Microbiological and Biochemical Origins of Human Axillary Odour." 83(2003):527-40.
6. M. Rahmati Roudsari, R. Karimi, S. Sohrabvandi and A. M. Mortazavian. 2015. "Health Effects of Probiotics on the Skin." Critical Reviews in Food Science and Nutrition 55(9):1219-40.
7. Julie Urban, Daniel J. Fergus, Amy M. Savage, Megan Ehlers, Holly L. Menninger, Robert R. Dunn and Julie E. Horvath. 2016. "The Effect of Habitual and Experimental Antiperspirant and Deodorant Product Use on the Armpit Microbiome." PeerJ 4:e1605.

### Summary of the invention

The preparation according to the discovery resolves the shortcoming given above for use in pharmacy, human and veterinary medicine, and cosmetics. The preparation contains a single-strain or multi-strain probiotic culture capable of surviving in vital form for a period of a number of months or years in an environment of highly concentrated alcohol. The probiotic culture may be advantageously stabilised, at least partially, by encapsulation in a layer of oil.

The expressions defined below are used in the text for the purposes of this discovery: *Stabilised dried vital probiotic culture:* a probiotic culture dehydrated, stabilised by, for example, drying with the use of lyophilisation, fluidised-bed drying or spray drying, preserving the vitality of the probiotic cells, which will begin to reproduce in a suitable environment (sufficient water and nutrients).
*Deodorant*/*perfume:* a solution applied in a spray to the body or to textiles primarily to restrict body odour caused by the bacterial decomposition of sweat.
*Perfuming ingredient:* fragrances, particularly natural extracts or synthetic essences with a strong aroma, that are a basic ingredient in the production of perfume and deodorants.

The subject of the invention is a composition for human or veterinary use as a deodorant, containing at least one probiotic culture in an amount of 1.10² to 1.10¹³ CFU (colony-forming units) in stabilised dried vital form to 1 g of the total weight of the composition or to 1 ml of the total volume of the composition and 90 % to 99.9999 % by weight of 98-99.9% C₁₋₆ alcohol. The C₁₋₆ alcohol is preferably ethanol.

A preferred amount of probiotic culture is 1x10³-1x10¹¹ CFU to 1 g of total composition weight. The probiotic culture is composed advantageously of bacteria of the genera Lactobacillus, Bifidobacterium, Streptococcus, Propionibacterium, Enterobacterium, Bacillus, Saccharomyces, Nitrosomonas, Lactococcus, Pedicoccus, Alcaligenes, Enterococcus, Micrococcus, Acinetobacter, Corynebacterium, Malassezia or other aerobic and/or microaerophilic and/or anaerobic bacteria and/or yeast, preferentially lactic acid yeast, naturally occurring on the skin of humans or animals and/or any other bacteria naturally occurring on the skin of humans or animals impacting the metabolism of aromatic substances with a beneficial action on the health of humans or animals, or mixtures of them.

It was found during the research that if a stabilised dried (lyophilised, fluidised-bed dried, spray dried) probiotic culture is mixed with food or cosmetic oil based on triacylglycerols, preferentially from the group rapeseed oil, olive oil, sunflower oil, avocado oil, coconut oil, linseed oil, castor oil, evening primrose oil, almond oil, argan oil, grapeseed oil, apricot oil, jojoba oil, bamboo butter, caraway oil, rose oil, raspberry seed oil, coffee oil and plum kernel oil, and subsequently mixed with a minimum of 98 % alcohol, preferentially ethanol, it is capable of maintaining long-term vitality.

The subject of the discovery is also a composition containing at least one probiotic culture in an amount of 1.10² to 1.10¹³ CFU in dried vital form to 1 g of the overall weight of the composition and 90 % to 99.9999 % by weight of 98-99.9% C₁₋₆ alcohol and at least 0.001 % by weight of oil component of the total weight of the probiotic culture in the composition. The oil component is in a weight ratio to the probiotic culture of 1:1000 or preferably 1:500.

The oil component is based on triacylglycerols, preferentially selected from the group comprised of rapeseed oil, olive oil, sunflower oil, avocado oil, coconut oil, linseed oil, castor oil, evening primrose oil, almond oil, argan oil, grapeseed oil, apricot oil, jojoba oil, bamboo butter, caraway oil, rose oil, coffee oil, plum kernel oil or mixtures of them.

The subject of the discovery is also the method of preparation of the composition according to the discovery, containing an oil component, with a dried vital prebiotic culture or a mixture of prebiotic cultures being mixed with an oil component in a mixture in the form of a homogenous paste or dense suspension, to which alcohol is added, the suspension is thoroughly mixed and additional ingredients, such as perfume essence, prebiotics, moisturisers, etc., are added to the resultant suspension.

The composition according to the invention may also contain a prebiotic component, advantageously chosen from a group comprised of fructooligosaccharides, galactooligosaccharides, glucooligosaccharides, mannans, xylans, sugar alcohols, pectin, inulin, dextrin, maltodextrin, glycogen, maize, potato and other starch, microbial proteins of plant or animal origin, and other physiologically acceptable proteins or mixtures of them.

The composition also advantageously contains a perfuming ingredient based on natural extracts or synthetic essential essences that make it pleasantly aromatic.

The subject of the discovery is also a preparation in the form of a spray deodorant stored in a vessel with an atomiser containing the composition according to the discovery and a propellant gas, preferably an inert gas from the group N₂, CO₂ and propane-butane or a mixture of these gases. These gases are advantageous for maintaining the viability of bacteria as, along with a minimum of 98 % alcohol, they paradoxically create an environment that maintains stabilised anhydrous bacteria viable over the long term. Antiperspirants are a subgroup of deodorants that additionally contain substances limiting sweating. The deodorant may, then, be fortified with ingredients restricting sweating.

The preparation may also contain vitamins, minerals, plant extracts and other physiologically beneficially substances to a maximum amount of 99 % by weight of the total weight of the composition.

The preparation according to the discovery will find applications in, for example, human and veterinary perfumes or sprays, medicinal agents, and cosmetic and hygiene products designed for application to the skin, hair, body hair, fur or textiles in the form of a spray.

### Figures

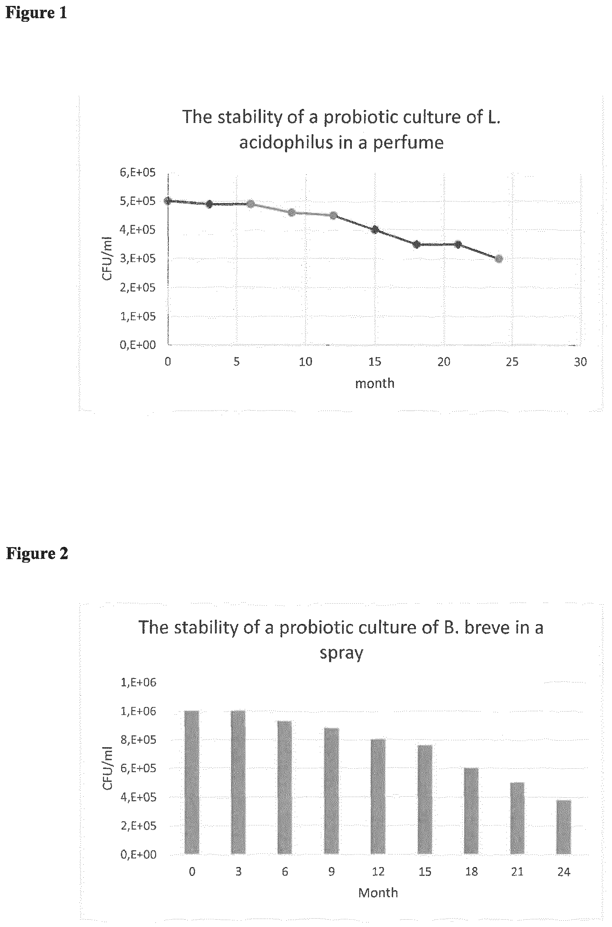

Figure 1: The stability of a lyophilised probiotic culture of *Lactobacillus acidophilus* in a deodorant in the form of a spray according to example 1, in which the probiotic culture is preserved in sunflower oil and 99 % ethanol in a pressure bottle with nitrogen as the propellant gas and perfuming ingredient A. The graph shows the dependency between the number of vital cultures of CFU/ml in nine samples of the preparation and time over a period of 24 months.
Figure 2: The stability of a lyophilised probiotic culture of *Bifidobacterium breve* in a deodorant in the form of a spray according to example 2, in which the probiotic culture is preserved in rapeseed oil and 98 % ethanol in a pressure bottle with a mixture of propane and butane as the propellant gas and perfuming ingredient B. The graph shows the dependency between the number of vital cultures of CFU/ml in nine samples of the preparation and time over a period of 24 months.

### Examples of the invention embodiments

### Example 1

100 g of a lyophilised probiotic culture of *Lactobacillus acidophilus* of a concentration of 150 billion CFU/g is mixed thoroughly with 100 g of sunflower oil until a homogenous paste is formed. This paste is then mixed in 10 litres of 99% ethanol. 1 % by weight of perfuming ingredient A is added to the suspension formed. The deodorant made in this way is filled in pressure bottles with an atomiser and pressurised with propellant gas - nitrogen.

### Stability study of the probiotic culture in the composition

1 ml of sample was taken in a sterile manner from the composition given in example 1 stored under the given conditions on nine occasions over the course of 24 months. During each collection, the sample was homogenised in 100 ml of MRS broth with cysteine in an Erlenmeyer flask.

A decimal dilution was then performed in test tubes - 1 ml of inoculum in 9 ml of MRS broth with cysteine to 10⁻⁶ and seeding of 100 µl of diluted inoculum on a large Petri disk on MRS agar with cysteine; each dilution was inoculated in three parallels.

The dishes with MRS agar were cultivated in a CO₂ thermostat for 48 hours at 37 °C; the number of CFU was then counted on each dish with a "countable" number of colonies and the average number of CFU calculated for the given composition sample (Figure 1).

It is clear from the graph that the average number of CFU/ml of probiotic culture of *Lactobacillus acidophilus* fell from the initial quantity of 5 x 10⁵ CFU/ml over the course of 24 months to 3 x 10⁵ CFU/ml, and that the results of analysis confirm that the culture maintains an extremely satisfactory vitality under the given conditions and is truly stable in the proposed composition.

### Example 2

1 kg of a lyophilised probiotic culture of *Bifidobacterium breve* of a concentration of 300 billion CFU/g was mixed thoroughly with 800 g of rapeseed oil until a homogenous paste was formed. This paste was then mixed in 200 litres of 98% ethanol. 1.5 % by weight of perfuming ingredient B was added to the suspension formed. The deodorant made in this way was filled in pressure bottles with an atomiser and pressurised with propellant gas - a mixture of propane and butane.

A stability study was conducted on the probiotic culture in the composition in the same way as in Example 1; the average number of CFU/ml of cultures in the composition over 24 months is depicted in the graph (Figure 2). It is clear from the graph that the average number of CFU/ml of the probiotic culture of *Bifidobacterium breve* fell from an initial quantity of 1 x 10⁶ CFU/ml over the course of 24 months to approximately 4 x 10⁵ CFU and that the results of analysis confirm that the culture maintains an extremely satisfactory vitality under the given conditions and is truly stable in the proposed composition.

### Example 3

500 g of a fluidised-bed dried probiotic culture of *Saccharomyces boulardii* of a concentration of 20 billion CFU/g was mixed thoroughly with 300 g of olive oil until a homogenous paste was formed. This paste was then mixed in 15 litres of 98.7% ethanol. 2 % by weight of a perfuming ingredient was added to the suspension formed. The deodorant made in this way was filled in pressure bottles with an atomiser and pressurised with propellant gas - a mixture of propane and butane.

### Example 4

100 g of a spray-dried probiotic culture of *Lactobacillus rhamnosus* of a concentration of 200 billion CFU/g was mixed thoroughly with 150 g of avocado oil until a homogenous paste was formed. This paste was then mixed in 20 litres of 99.2% ethanol. 2 % by weight of a perfuming ingredient was added to the suspension formed. The deodorant made in this way was filled in pressure bottles with an atomiser and pressurised with propellant gas - CO₂.

### Example 5

100 g of a spray-dried probiotic culture of *Lactobacillus rhamnosus* of a concentration of 200 billion CFU/g was mixed thoroughly with 150 g of avocado oil until a homogenous paste was formed. This paste was then mixed in 20 litres of 99.2% ethanol. 2 % by weight of a perfuming ingredient was added to the suspension formed. The deodorant made in this way was filled in pressure bottles with an atomiser and pressurised with propellant gas - CO₂.

### Example 6

100 g of a spray-dried probiotic culture of *Pediococcus pentosaceus* of a concentration of 100 billion CFU/g was mixed thoroughly with 150 g of avocado oil until a homogenous paste was formed. This paste was then mixed in 20 litres of 99.2% ethanol. 1.8 % by weight of a perfuming ingredient was added to the suspension formed. The deodorant made in this way was filled in pressure bottles with an atomiser and pressurised with propellant gas - CO₂.

### Example 7

10 kg of a lyophilised probiotic culture of *Streptococcus thermophilus* of a concentration of 200 billion CFU/g was mixed thoroughly with 6 kg of rapeseed oil until a homogenous paste was formed. This paste was then mixed in 500 litres of 99% ethanol. 2.3 % by weight of a perfuming ingredient and 1 kg of chicory inulin was added to the suspension formed. The deodorant made in this way was filled in pressure bottles with an atomiser and pressurised with propellant gas - N₂:CO₂ at a ratio of 9:1.

### Example 8

1 kg of a mixture of lyophilised probiotic cultures of *Streptococcus thermophilus, Lactobacillus plantarum* and *Saccharomyces cerevisiae* at a ratio of 3:6:1 of a concentration of 200 billion CFU/g was mixed thoroughly in 300 litres of 99.2% ethanol. 1.4 % by weight of a perfuming ingredient and 1 kg of mannitol was added to the suspension formed. The deodorant made in this way was filled in aluminium pressure bottles of a volume of 50 ml with an atomiser and pressurised with propellant gas - N₂.

### Example 9

10 kg of a lyophilised probiotic culture of *Bacillus coagulans* of a concentration of 15 billion CFU/g was mixed thoroughly with 2 kg of rapeseed oil until a homogenous paste was formed. This mixture was then mixed in 300 litres of 96% ethanol. 1.5 % by weight of a perfuming ingredient was added to the suspension formed. The deodorant made in this way was filled in pressure bottles with an atomiser containing a metal ball to shake up any precipitate before application of the spray and pressurised with propellant gas - N₂.

## Claims

1. A composition containing at least one probiotic culture for human or veterinary use as a deodorant, wherein the probiotic culture is a bacteria and/or yeast, and/or any other bacteria naturally occurring on the skin of humans or animals, and/or any other bacteria naturally occurring on the skin of humans or animals impacting the metabolism of aromatic substances, in dried vital form, in an amount of 1.10² to 1.10¹³ CFU to 1 g of the total weight of the composition and 90 % to 99.9999 % by weight of 98-99.9% C₁₋₆ alcohol of the total weight of the composition.

2. The composition of claim 1, wherein an advantageous amount of probiotic culture is 1x10³-1x10¹¹ CFU to 1 g of the total weight of the composition.

3. The composition of claims 1 to 2, wherein the probiotic culture is formed of bacteria of the genera Lactobacillus, Bifidobacterium, Streptococcus, Propionibacterium, Enterobacterium, Bacillus, Saccharomyces, Nitrosomonas, Lactococcus, Pedicoccus, Alcaligenes, Enterococcus, Micrococcus, Acinetobacter, Corynebacterium and Malassezia.

4. The composition of claims 1 to 3, wherein the probiotic culture is lactic acid bacteria.

5. The composition of claims 1 to 4, wherein the C₁₋₆ alcohol is ethanol.

6. The composition of claims 1 to 5 containing at least one probiotic culture in an amount of 1.10² to 1.10¹³ CFU in dried vital form to 1 g of the total weight of the composition and 90 % to 99.9999 % by weight of 98-99.9% C₁₋₆ alcohol and at least 0.001 % by weight of an oil component of the total weight of the probiotic culture in the composition.

7. The composition of claim 6, wherein the oil component is in a weight ratio to the probiotic culture of 1:1000, preferably 1:500.

8. The composition of claims 6 to 7, wherein the oil component is based on triacylglycerols, preferentially selected from the group rapeseed oil, olive oil, sunflower oil, avocado oil, coconut oil, linseed oil, castor oil, evening primrose oil, almond oil, argan oil, grapeseed oil, apricot oil, jojoba oil, bamboo butter, caraway oil, rose oil, coffee oil and plum kernel oil.

9. The composition of claims 1 to 8 also containing a prebiotic component.

10. The composition of claim 9, wherein the prebiotic component is a component selected from a group comprised of fructooligosaccharides, galactooligosaccharides, glucooligosaccharides, mannans, xylans and sugar alcohols.

11. The composition of claims 1 to 10 also containing a perfuming ingredient.

12. The method of preparation of the composition of claims 6 to 11, in which a dried vital probiotic culture or mixture of probiotic cultures is mixed with an oil component in a mixture in the form of a homogenous paste or dense suspension, to which alcohol is then added, the suspension thoroughly mixed, and further ingredients added to the resultant suspension.

13. A preparation containing the composition of claims 1 to 11 for use as a deodorant in a spray, wherein the preparation contains a propellant gas.

14. The preparation of claim 13, wherein the propellant gas is N₂, CO₂, propane, butane or a mixture of these gases.
